# EUROPEAN PATENT APPLICATION

(11) **EP 3 196 289 A1**
(43) Date of publication of application: **26.07.2017**
(21) Application number: 15842148.7
(22) Date of filing: 09.09.2015
(51) Int. Cl.: C12M 1/00, C12M 3/02, C12N 5/00

(54) **LIQUID DELIVERY METHOD FOR CELL CULTURE SYSTEM, AND CELL CULTURE SYSTEM**

(30) Priority: 17.09.2014 JP 2014189272
(71) Applicant: Toyo Seikan Group Holdings, Ltd., Shinagawa-ku Tokyo 141-8627 (JP)
(72) Inventor: KOSEKI, Osamu, Tokyo 141-8627 (JP); SUENAGA, Ryo, Yokohama-shi Kanagawa 240-0062 (JP); KASHIWABARA, Ken, Yokohama-shi Kanagawa 240-0062 (JP)
(74) Representative: Liebetanz, Michael
(86) International application number: PCT/JP2015/004587
(87) International publication number: WO 2016/042741

(57) **Abstract**

A cell culture system disclosed herein comprises a culture medium container (10) to supply a culture medium to a culture container, two culture containers (20, 30) for use in cell culture, and a transfer tube (40) to connect these containers, whereby liquid delivery can be performed by use of only one pump (41). The containers in the cell culture system are connected to the transfer tube (40) in the order of the culture medium container (10), the second culture container (30) and the first culture container (20); the only one pump (41) is attached to the transfer tube; the second culture container is connected to the transfer tube on both sides of the pump by culture container connecting tubes (42) to connect the second culture container and the transfer tube, to inject cells into the first culture container; the culture medium is transferred from the culture medium container to the first culture container by the pump; after the cell culture in the first culture container, a cell suspension is transferred from the first culture container to the second culture container; and the culture medium is transferred from the culture medium container to the second culture container.

## Description

### FIELD

The present invention relates to a cell culture system, and more particularly, it relates to a liquid delivery method for the cell culture system.

### BACKGROUND

In recent years, it has been required that cells, tissues or the like are efficiently cultured in large amounts under an artificial environment in fields of production of medicines, gene therapy, regenerative therapy, immunotherapy and the like.

In such cell culture, a culture medium container to supply a culture medium to a culture container and the culture container for use in cell culture are connected by a tube to constitute a cell culture system, and the cell culture is performed in a closed system by use of this cell culture system, thereby avoiding a risk of contamination to proliferate the cells.

In this cell culture system, the culture medium is generally required to be refrigerated, and hence the culture medium container is stored in a refrigerating chamber. Furthermore, in the cell culture, it is necessary to control a temperature, air environment and the like, and hence the culture container is stored in an incubator. Then, the culture medium is transferred from the culture medium container to the culture container via the tube.

Alternatively, in the cell culture, there is a case where a plurality of culture containers may be used and a cell suspension may be transferred between the culture containers in accordance with a culture situation of the cells.

It is general that the transfer of a content between the containers is performed by using a pump attached to the tube.

In the case of the cell culture system in which the culture medium container and the plurality of culture containers are connected by the tubes, it has heretofore been necessary to perform the transfer of the content by use of a plurality of pumps.

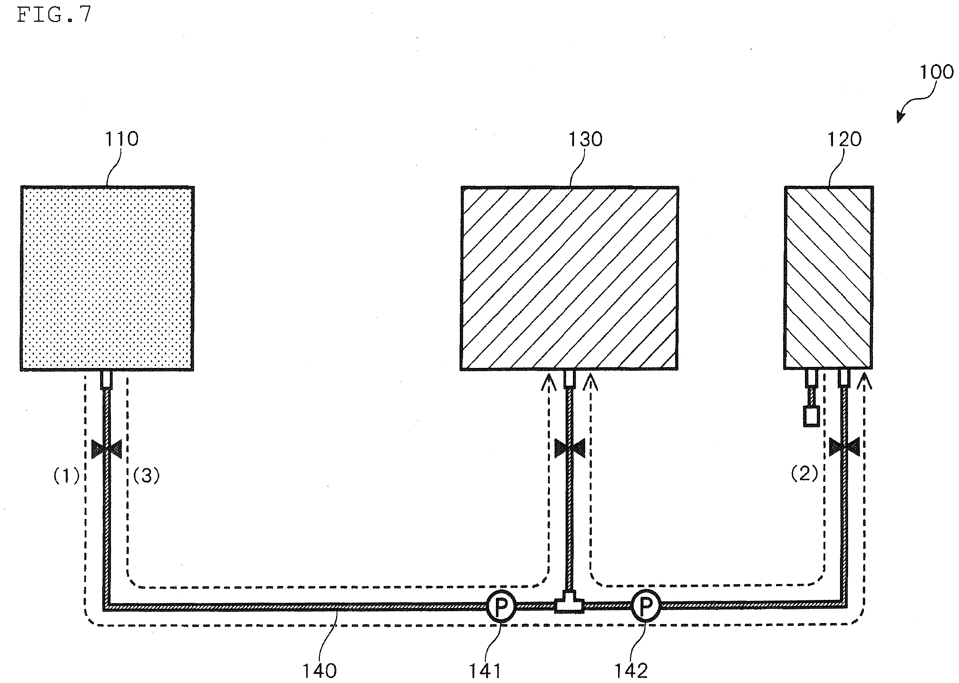

For example, in an example of FIG 7, a culture medium container 110, a first culture container 120 in which the cell culture is first performed and a second culture container 130 in which the cell culture is next performed are connected by using a transfer tube 140, a first pump 141 is interposed between the culture medium container 110 and the second culture container 130, and a second pump 142 is interposed between the second culture container 130 and the first culture container 120. Successively, the cells are injected into the first culture container 120, and then a culture medium is transferred from the culture medium container 110 to the first culture container 120 via the pump 141 and the pump 142. After the cell culture in the first culture container 120, a cell suspension is next transferred from the first culture container 120 to the second culture container 130 via the pump 142, and then the culture medium is transferred from the culture medium container 110 to the second culture container 130 via the pump 141.

Additionally, as the pumps, tube roller pumps are suitably used to constitute the cell culture system as the closed system. In a case where such pumps are used, it is necessary to simultaneously operate the pump 141 and the pump 142 in the above-mentioned transfer of the culture medium from the culture medium container 110 to the first culture container 120.

However, for the reason that inner diameters of the tubes are not even, and for other reasons, it is difficult to completely synchronize these pumps connected in series, and there has been the problem that a transfer efficiency of the culture medium is low.

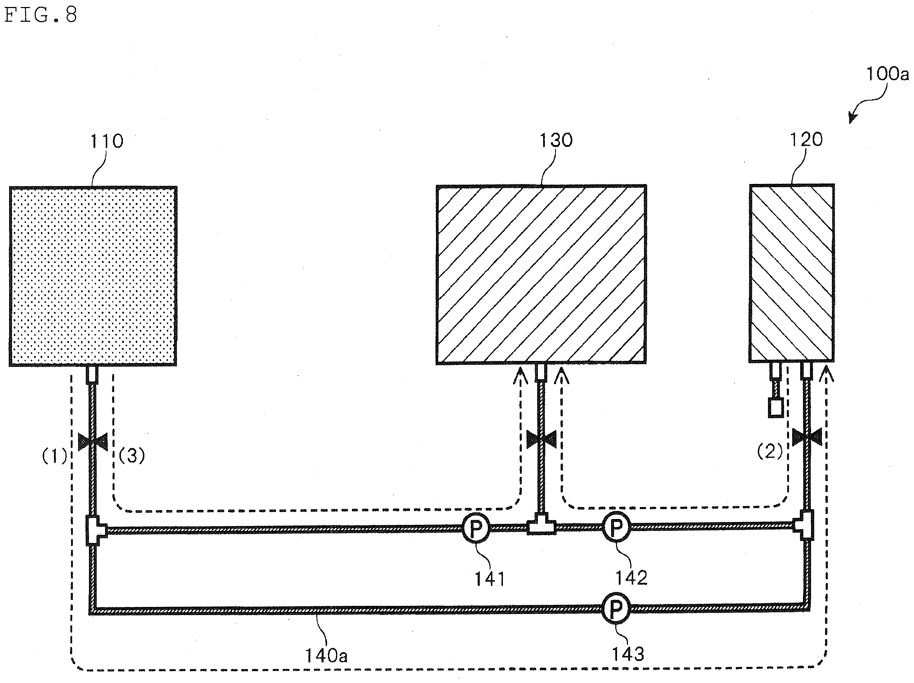

On the other hand, as shown in FIG. 8, there is added a tube constituting a new path to transfer the culture medium from the culture medium container 110 to the first culture container 120, and a third pump 143 is attached to this tube, thereby making it possible to solve the above problem.

However, the pump is more expensive than a valve member or the like, and the attachment of the pump to the tube is a laborious operation. Therefore, it is desirable to decrease the number of the pumps in the cell culture system as much as possible.

Here, in the cell culture system comprising the culture medium container, the culture container and others, an example of a technology of transferring the culture medium or the cell suspension is a cell culture system described in Patent Literature 1 (a cell culture kit in the same literature). In this cell culture system, it is possible to efficiently perform transfer of a content between containers in a closed system by use of the number of pumps which corresponds to the number of ports of the culture container.

Patent Literature 1: WO 2013/114845

### SUMMARY

### TECHNICAL PROBLEM

However, a cell culture system described in Patent Literature 1 does not improve a liquid delivery efficiency in a case of using a plurality of culture containers, and also is not capable of decreasing the number of pumps.

The present invention has been developed in view of the above circumstances, and an object thereof is to provide a liquid delivery method in which synchronization of pumps connected in series is not required and a laborious operation of attaching the pump to a tube is minimized in a cell culture system comprising a culture medium container to supply a culture medium to a culture container, two culture containers for use in cell culture, and a transfer tube to connect these containers, and another object is to provide the cell culture system.

### SOLUTION TO PROBLEM

A liquid delivery method for a cell culture system according to the present invention is a liquid delivery method for a cell culture system comprising a culture medium container to supply a culture medium to a culture container, a first culture container for use in cell culture, a second culture container for use in cell culture, and a transfer tube to connect these containers, the method comprising: connecting the respective containers in the cell culture system to the transfer tube in the order of the culture medium container, the second culture container and the first culture container; attaching only one pump to the transfer tube; connecting the second culture container to the transfer tube on both sides of the pump by culture container connecting tubes to connect the second culture container and the transfer tube; injecting cells into the first culture container; transferring the culture medium from the culture medium container to the first culture container by the pump; and after the cell culture in the first culture container, transferring a cell suspension from the first culture container to the second culture container; and transferring the culture medium from the culture medium container to the second culture container.

Furthermore, the cell culture system according to the present invention is a cell culture system comprising a culture medium container to supply a culture medium to a culture container, a second culture container for use in cell culture, a first culture container for use in cell culture, and a transfer tube to connect these containers, wherein the respective containers in the cell culture system are connected to the transfer tube in the order of the culture medium container, the second culture container and the first culture container, the transfer tube comprises only one pump, and the second culture container is connected to the transfer tube on both sides of the pump in the transfer tube by culture container connecting tubes to connect the second culture container and the transfer tube.

### ADVANTAGEOUS EFFECTS OF INVENTION

According to the present invention, in a cell culture system comprising a culture medium container to supply a culture medium to a culture container, two culture containers for use in cell culture, and a transfer tube to connect these containers, liquid delivery can be performed by use of only one pump. Therefore, an inexpensive system is obtainable in which synchronization of pumps is not required and a laborious operation of attaching the pump to the tube is minimized.

### BRIEF DESCRIPTION OF THE DRAWINGS

FIG 1 is an explanatory view showing an outline of a cell culture system according to a first embodiment of the present invention;
FIG 2 is an explanatory view showing a liquid delivery procedure in the cell culture system according to the first embodiment of the present invention;
FIG 3 is an explanatory view showing an outline of a cell culture system according to a second embodiment of the present invention;
FIG. 4 is an explanatory view showing an outline of a cell culture system according to a third embodiment of the present invention;
FIG 5 is an explanatory view showing a liquid delivery procedure in the cell culture system according to the third embodiment of the present invention;
FIG. 6 is an explanatory view showing an outline of a cell culture system according to a fourth embodiment of the present invention;
FIG 7 is an explanatory view showing an outline of a conventional cell culture system (1); and
FIG 8 is an explanatory view showing an outline of a conventional cell culture system (2).

### DETAILED DESCRIPTION

Hereinafter, a liquid delivery method for a cell culture system according to an embodiment of the present invention and preferable embodiments of the cell culture system will be described with reference to the drawings.

### [First Embodiment]

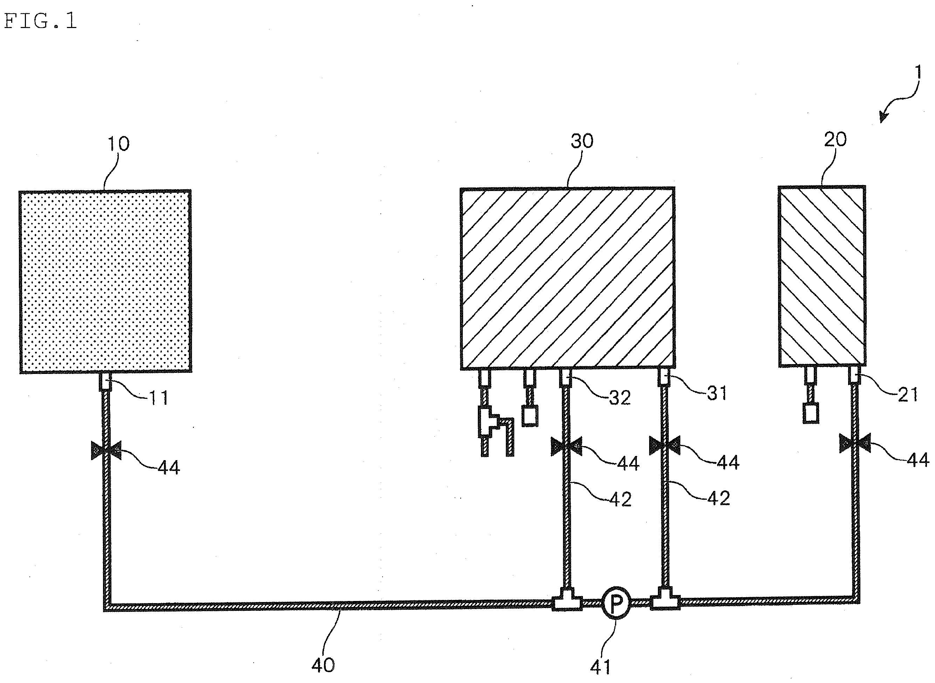

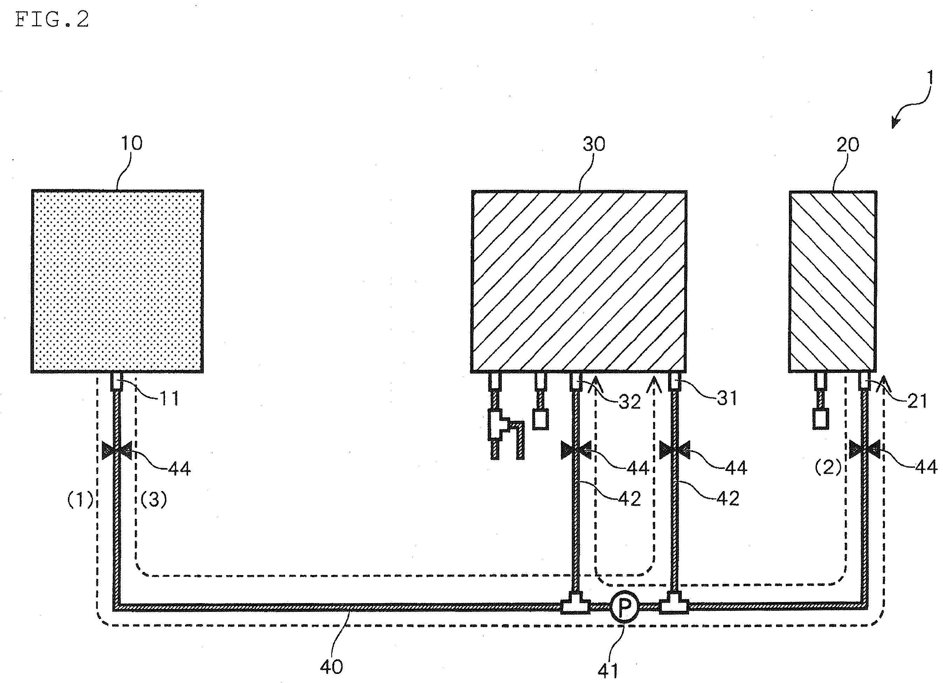

First, a liquid delivery method for a cell culture system according to a first embodiment of the present invention and the cell culture system will be described with reference to FIG. 1 and FIG. 2.

As shown in FIG. 1, the cell culture system of the present embodiment comprises a culture medium container 10 to supply a culture medium to a culture container, a first culture container 20 and a second culture container 30 for use in cell culture, and a transfer tube 40 to connect these containers.

The first culture container 20 is a culture container into which cells are injected, to first perform cell culture, and is suitably usable as, for example, an activating culture container for use in the cell culture to activate the cells. In a case of activating floating cells such as lymphocytes by use of the first culture container 20 as the activating culture container, a substance such as an anti-CD3 antibody to activate the cells is immobilized in a solid phase on a bottom surface in the first culture container 20.

The second culture container 30 is a container into which, after the cell culture in the first culture container, a cell suspension in the first culture container is transferred, to perform the cell culture, and the second culture container is suitably usable as, for example, a proliferating culture container for use in the cell culture to proliferate the cells.

In this way, the first culture container 20 is used as the activating culture container, and the second culture container 30 is used as the proliferating culture container, thereby making it possible to suitably proliferate floating cells such as the lymphocytes.

In the cell culture system of the present embodiment, these containers are connected to the transfer tube 40 in the order of the culture medium container 10, the second culture container 30 and the first culture container 20. It is to be noted that in a case where the containers are seen from a back side, the containers are connected to the transfer tube 40 in the order of the first culture container 20, the second culture container 30 and the culture medium container 10, and these orders are the same.

The culture medium container 10 comprises a port 11 for liquid delivery (including inward delivery and outward delivery), and is connected to the transfer tube 40 in the port 11. It is to be noted that in the cell culture system of the present embodiment, there is not any special restriction on the number of the culture medium containers 10 to be connected, and at least one culture medium container 10 can be connected to the transfer tube 40 to constitute the cell culture system of the present embodiment.

The first culture container 20 comprises a port 21 for liquid delivery, and is connected to the transfer tube 40 in the port 21.

As shown in FIG. 1, it is preferable that the port 21 is disposed on the side of the transfer tube 40 in a peripheral edge portion of the first culture container 20 and most apart from the second culture container 30. In this case, in delivering a content out from the first culture container 20, i.e., in transferring the cell suspension from the first culture container 20 to the second culture container 30, the cell culture system is tilted so as to dispose the port 21 downwardly, thereby making it possible to inhibit the cell suspension from remaining in the first culture container 20.

The second culture container 30 comprises a port 31 and a port 32 for liquid delivery, one side end portions of two culture container connecting tubes 42 are connected to these ports respectively, and the other side end portions are connected to the transfer tube 40. A pump 41 is attached to a portion between connected portions of the culture container connecting tubes 42 in the transfer tube 40.

That is, the cell culture system according to the present embodiment comprises only one pump, and has a constitution in which the second culture container 30 is connected to the transfer tube 40 on both sides of the pump 41 by the culture container connecting tubes 42 to connect the second culture container 30 and the transfer tube 40.

As shown in FIG. 1, it is preferable that the port 31 is disposed on the side of the transfer tube 40 in a peripheral edge portion of the second culture container 30 and most apart from the culture medium container 10. In this case, in delivering the content out from the second culture container 30, i.e., in transferring and collecting the cell suspension from the second culture container 30 to the culture medium container 10, the cell culture system is tilted so as to dispose the port 31 downwardly, thereby making it possible to inhibit the cell suspension from remaining in the second culture container 30.

There is not any special restriction on the number of the ports in each of these containers, and the number of the ports can be adjusted to be optional. In an example of FIG 1, the first culture container 20 comprises a port for cell injection in addition to the port 21. Furthermore, the second culture container 30 comprises a preliminary port to inject a proliferation factor or the like and a sampling port in addition to the ports 31 and 32.

When the liquid delivery is not performed between the containers, a flow channel from each container is closed with a closing member such as a clip. That is, in the example of FIG. 1, the flow channel between each container and the pump 41 is stopped with the clip. Further, in performing the liquid delivery between the containers, the closing member for the two containers between which the liquid delivery is performed is only removed to open the flow channel, and the liquid delivery by the pump 41 is performed. It is to be noted that the positions of the closing members and the number thereof are not limited to those shown in FIG 1 and can suitably be set, for example, by disposing the closing members on the side of the pump 41.

In general, the culture medium container 10 having gas barrier properties to oxygen and carbon dioxide is used so that a pH of the stored culture medium does not noticeably change in a culture period. For the purpose of avoiding the problem that a high-concentration carbonic acid gas included in the culture medium falls out in the air and that the concentration of the carbonic acid gas in the culture medium decreases to increase the pH, it is desirable to minimize leaking of carbon dioxide out from the culture medium container 10 as much as possible, and it is also desirable to prevent oxidation of the culture medium.

The first culture container 20 and the second culture container 30 are formed into a bag shape by use of a soft packaging member as a material, and partially or entirely have transparency so that contents can be confirmed. Furthermore, these culture containers are required to have a gas permeability (an oxygen and carbon dioxide permeability) required for cell culture, and are preferably used at 37°C under a culture environment where a concentration of carbon dioxide is 5%. Furthermore, it is preferable that the culture containers have low cytotoxicity, low elution, and radiation sterilization suitability to achieve a high cell proliferating efficiency.

As a material of the culture container which satisfies these conditions, a polyethylene-based resin is preferable. Examples of this polyethylene-based resin include polyethylene, a copolymer of ethylene and α-olefin, a copolymer of ethylene and vinyl acetate, and ionomers in which ethylene, an acrylic acid or methacrylic acid copolymer and metal ions are used. Furthermore, polyolefin, a styrene-based elastomer, a polyester-based thermoplastic elastomer, a silicone-based thermoplastic elastomer, a silicone resin or the like is usable.

There is not any special restriction on a shape of each of the culture medium container 10, the first culture container 20 and the second culture container 30 and a shape of a containing section of the container, and in the example of FIG. 1, the container is formed into a rectangular shape. The container can be manufactured by sealing four sides of the container with a heat seal, or can be manufactured as an integrally formed bag by blow forming.

It is preferable that each port is disposed on the transfer tube 40 side, and it is also preferable that the containing section gradually narrows toward an attaching portion of the tube.

A material of the transfer tube 40 and the culture container connecting tubes 42 may suitably be selected in accordance with a use environment, but is preferably excellent in gas permeability. For example, there is usable a silicone rubber, a soft vinyl chloride resin, a polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, or the like. Furthermore, as the styrene-based elastomer, there is usable, for example, SBS (styrene-butadienestyrene), SIS (styrene-isoprene-styrene), SEBS (styrene-ethylene-butylene-styrene), SEPS (styrene-ethylene-propylene-styrene), or the like.

In FIG. 1, for example, a silicone tube is used in a portion of the transfer tube 40 to which the pump 41 is attached, and a soft vinyl chloride resin tube is suitably usable in another portion thereof.

Furthermore, connection between the tubes in the transfer tube 40 can be performed with a luer connector or another coupling means. Furthermore, in the transfer tube 40, flow channel opening/closing means such as a pinch valve, a two-way stopcock or a three-way stopcock may be disposed.

There is not any special restriction on a type of pump 41, but a tube roller pump such as a peristaltic pump (R) is suitably usable because a closed system in the cell culture system is easy to be realized.

It is to be noted that although not shown, it is preferable that the cell culture system comprises an observation device to observe states of the cells or the culture medium in each culture container. For example, the observation device comprising, for example, a CCD camera, an objective lens, illumination, an information processing unit and others is usable. In this case, the culture container mounted horizontally on a mounting base having an observation hole is illuminated with light from the upside, and the cells in the culture container or the like can automatically be photographed at a predetermined timing with the CCD camera and the objective lens from the downside of the observation hole. Further, obtained image data is transmitted to the information processing unit, and the image data is analyzed in the information processing unit, thereby making it possible to calculate the number of the cells in the culture container, a cell density, or the like.

By the cell culture method for the cell culture system of the present embodiment, the cells are first injected into the first culture container 20 by use of the above-mentioned cell culture system.

Next, the pump 41 is operated, and by the pump 41, the culture medium is transferred from the culture medium container 10 to the first culture container 20 as shown in FIG. 2 (an arrow (1) in the same figure). Further, in the first culture container 20, the cell culture is performed for a predetermined period.

After the cell culture in the first culture container 20, the cell suspension is transferred from the first culture container 20 to the second culture container 30 by the pump 41 (an arrow (2) in the same figure), and next the culture medium is transferred from the culture medium container 10 to the second culture container 30 (an arrow (3) in the same figure).

At this time, the transfer of the cell suspension from the first culture container 20 to the second culture container 30 is performed via the culture container connecting tube 42 connected to the transfer tube 40 on a culture medium container 10 side of the pump 41.

Furthermore, the transfer of the culture medium from the culture medium container 10 to the second culture container 30 is performed via the culture container connecting tube 42 connected to the transfer tube 40 on a first culture container 20 side of the pump 41.

In this way, according to the liquid delivery method for the cell culture system of the present embodiment, and the cell culture system, it is possible to appropriately perform, by use of only one pump 41, transfers of three systems, i.e., the transfer of the culture medium from the culture medium container 10 to the first culture container 20, the transfer of the cell suspension from the first culture container 20 to the second culture container 30 and the transfer of the culture medium from the culture medium container 10 to the second culture container 30. In consequence, it is possible to inexpensively manufacture the cell culture system, and it is also possible to miniaturize and manufacture the cell culture system.

### [Second Embodiment]

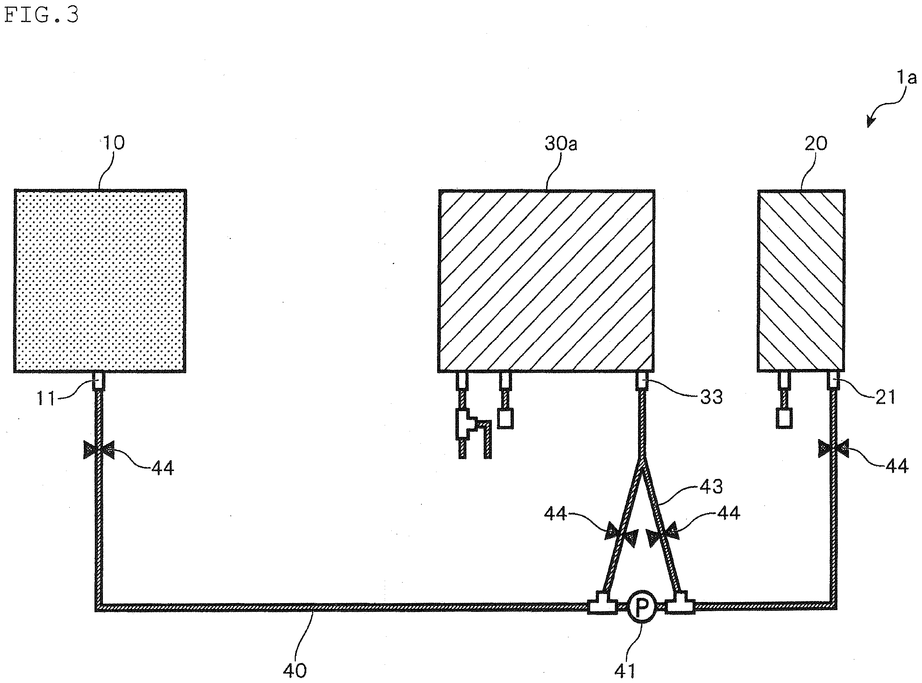

Next, a liquid delivery method for a cell culture system according to a second embodiment of the present invention and the cell culture system will be described with reference to FIG. 3.

As shown in FIG. 3, the cell culture system of the present embodiment is different from the first embodiment in that a second culture container 30a is connected to a transfer tube 40 by use of a culture container connecting tube 43 different in shape from the first embodiment. The other respect is similar to the first embodiment.

The second culture container 30a comprises a port 33 for liquid delivery, one end portion of the culture container connecting tube 43 of a shape branched into two ways is connected to this port, and the other two end portions thereof are connected to the transfer tube 40. A pump 41 is attached to a portion between connected portions of the culture container connecting tube 43 in the transfer tube 40.

That is, the cell culture system according to the present embodiment also comprises only one pump, and has a constitution in which the second culture container 30a is connected to the transfer tube 40 on both sides of the pump 41 by the culture container connecting tube 43 to connect the second culture container 30a and the transfer tube 40.

Further, transfer of a cell suspension from a first culture container 20 to the second culture container 30a is performed via a flow channel of the culture container connecting tube 43 connected to the transfer tube 40 on a culture medium container 10 side of the pump 41.

Furthermore, transfer of a culture medium from the culture medium container 10 to the second culture container 30a is performed via a flow channel of the culture container connecting tube 43 connected to the transfer tube 40 on a first culture container 20 side of the pump 41.

The other respect is similar to the first embodiment, and also according to the liquid delivery method for the cell culture system of the present embodiment and the cell culture system, it is possible to inexpensively minimize and manufacture a cell culture system by use of only one pump 41.

### [Third Embodiment]

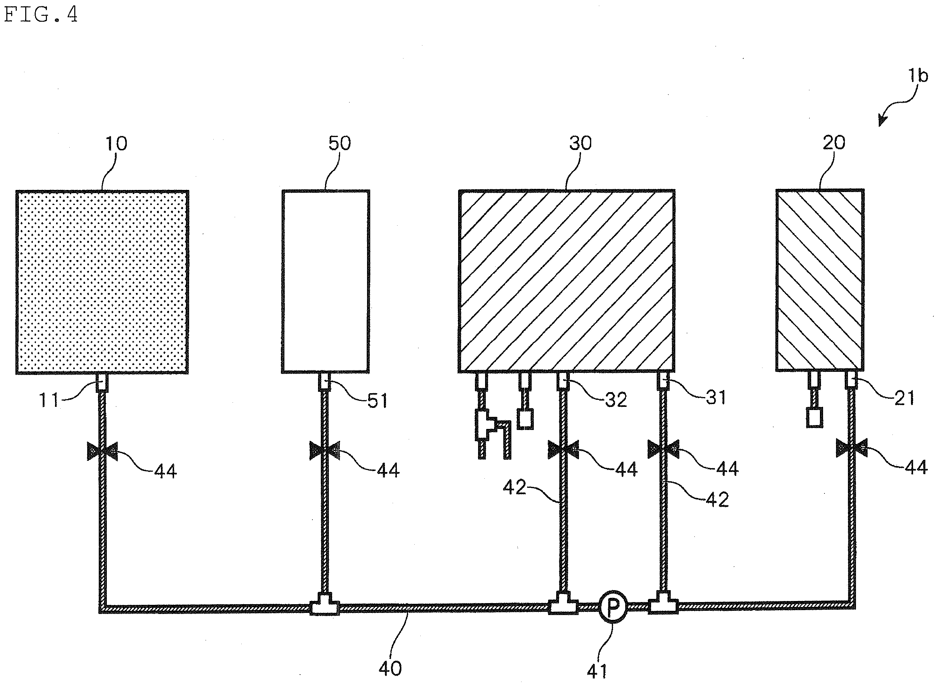

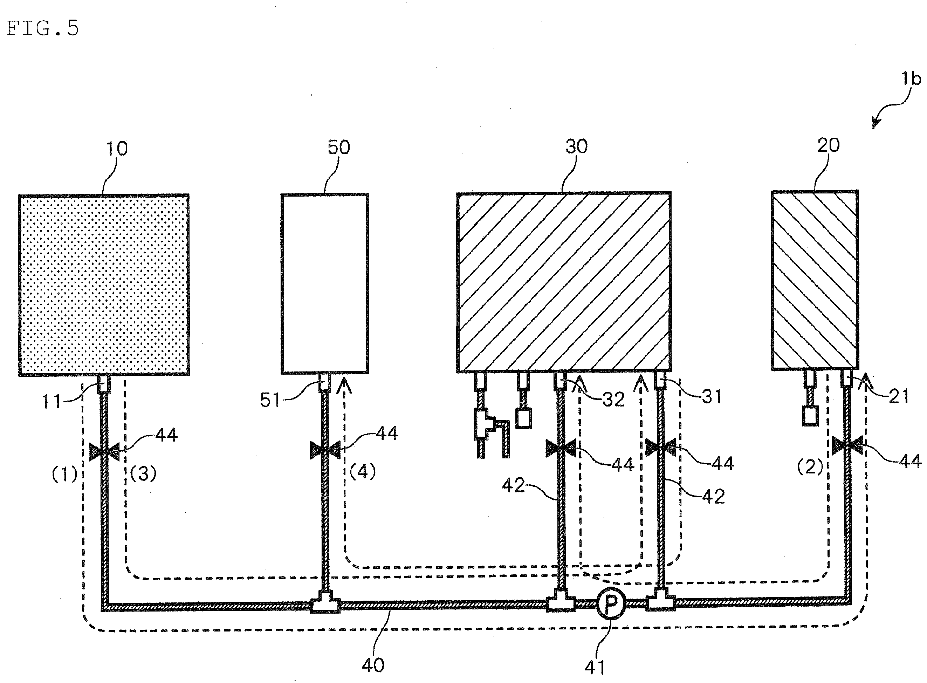

Next, there will be described a liquid delivery method for a cell culture system according to a third embodiment of the present invention, and the cell culture system with reference to FIG 4 and FIG 5.

As shown in FIG. 4, the cell culture system of the present embodiment is different from the first embodiment in that a collecting container 50 is stored. The other respect is similar to the first embodiment.

That is, in the cell culture system of the present embodiment, the collecting container 50 is connected to a transfer tube 40. In an example of FIG. 4, only one collecting container 50 is stored, but two or more collecting containers may be stored.

The collecting container 50 is a container to collect a cell suspension from a second culture container 30 after cell culture in the second culture container 30 is ended.

The collecting container 50 comprises a port 51 for liquid delivery, and is connected to the transfer tube 40 in the port 51.

The liquid delivery method for the cell culture system of the present embodiment is similar to the first embodiment from injection of cells into a first culture container 20 to transfer of a culture medium to the second culture container 30, and furthermore, transfer of the cell suspension from the second culture container 30 to the collecting container 50 is performed.

That is, a pump 41 is operated, and as shown in FIG 5, by the pump 41, the culture medium is transferred from a culture medium container 10 to the first culture container 20 (an arrow (1) in the same figure). Then, in the first culture container 20, cell culture is performed for a predetermined period.

After the cell culture in the first culture container 20, the cell suspension is transferred from the first culture container 20 to the second culture container 30 by the pump 41 (an arrow (2) in the same figure), and next the culture medium is transferred from the culture medium container 10 to the second culture container 30 (an arrow (3) in the same figure).

Furthermore, after cell culture in the second culture container 30 is ended, the cell suspension is transferred from the second culture container 30 to the collecting container 50 by the pump 41 (an arrow (4) in the same figure).

At this time, the transfer of the cell suspension from the second culture container 30 to the collecting container 50 is performed via a culture container connecting tube 42 connected to the transfer tube 40 on a first culture container 20 side of the pump 41.

According to the liquid delivery method for the cell culture system of the present embodiment and the cell culture system, by use of only one pump 41, it is possible to appropriately perform transfers of four systems, i.e., the transfer of the culture medium from the culture medium container 10 to the first culture container 20, the transfer of the cell suspension from the first culture container 20 to the second culture container 30, the transfer of the culture medium from the culture medium container 10 to the second culture container 30, and the transfer of the cell suspension from the second culture container 30 to the collecting container 50.

### [Fourth Embodiment]

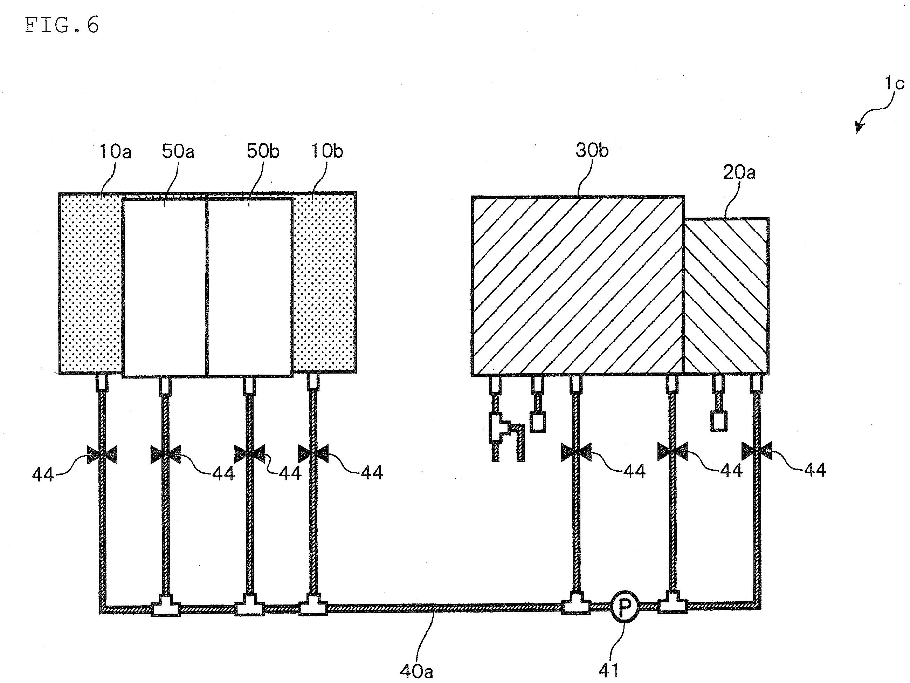

Next, a liquid delivery method for a cell culture system according to a fourth embodiment of the present invention and the cell culture system will be described with reference to FIG. 6.

As shown in FIG. 6, the cell culture system of the present embodiment is different from the third embodiment in that the system comprises two culture medium containers 10a and 10b and two collecting containers 50a and 50b. The other respect is similar to the third embodiment.

That is, in the cell culture system of the present embodiment, the two culture medium containers 10a and 10b are connected to a transfer tube 40a. In an example of FIG. 6, the culture medium containers 10a and 10b are integrally formed, but may be constituted separately. Furthermore, the cell culture system of the present embodiment may comprise three or more culture medium containers. These culture medium containers may be charged with the same culture medium or different culture mediums.

Furthermore, in the cell culture system of the present embodiment, the two collecting containers 50a and 50b are connected to the transfer tube 40a. In the example of FIG. 6, the collecting containers 50a and 50b are integrally formed, but may be constituted separately. Furthermore, the cell culture system of the present embodiment may comprise three or more collecting containers.

Furthermore, in FIG. 6, a first culture container 20a and a second culture container 30b are integrally formed, but may be constituted separately.

According to the liquid delivery method for the cell culture system of the present embodiment and the cell culture system, similarly to the third embodiment, it is possible to appropriately perform transfers of four systems (there are further more patterns of systems when each of two culture medium containers and two collecting containers is taken into consideration) by use of only one pump 41. Furthermore, the cell culture system comprises a plurality of culture medium containers, and hence it is possible to transfer different types of culture mediums to the culture container in accordance with types of cells or a culture situation.

The preferable embodiments of the present invention have been illustrated and described above, but the present invention is not limited to the above-mentioned embodiments, and needless to say, various changes can be implemented in the scope of the present invention.

For example, in the above-mentioned embodiments, there has been described the constitution in which the culture medium container, the culture container and the collecting container are connected to the transfer tube, but in culture of; for example, adhesive cells, an enzyme solution supply container for supply of an enzyme to peel the cells from a container wall, an inhibitor solution supply container for supply of an inhibitor to inhibit activity of this enzyme and another container are further connected, thereby making it possible to transfer the enzyme or the inhibitor to the culture container in the same manner as in the culture medium. Furthermore, the cell culture system of the third embodiment or the fourth embodiment can suitably be changed by using, for example, a culture container connecting tube of a shape branched into two ways in the second embodiment.

### INDUSTRIAL APPLICABILITY

The present invention is suitably utilizable in a regenerative therapy, immunotherapy, antibody medicine production or the like to culture a large amount of cells in a closed system by use of culture containers of the cells.

### REFERENCE SIGNS LIST

- 1, 1a, 1 b and 1 c: cell culture system
- 10, 10a and 10b: culture medium container
- 11: port
- 20 and 20a: first culture container
- 21: port
- 30, 30a and 30b: second culture container
- 31, 32 and 33: port
- 40 and 40a: transfer tube
- 41: pump
- 42 and 43: culture container connecting tube
- 44: closing member
- 50, 50a and 50b: collecting container
- 51: port

## Claims

1. A liquid delivery method for a cell culture system comprising a culture medium container to supply a culture medium to a culture container, a first culture container for use in cell culture, a second culture container for use in cell culture, and a transfer tube to connect these containers, the method comprising:
connecting the respective containers in the cell culture system to the transfer tube in the order of the culture medium container, the second culture container and the first culture container;
attaching only one pump to the transfer tube;
connecting the second culture container to the transfer tube on both sides of the pump by culture container connecting tubes to connect the second culture container and the transfer tube;
injecting cells into the first culture container; and
transferring the culture medium from the culture medium container to the first culture container by the pump, and after the cell culture in the first culture container, transferring a cell suspension from the first culture container to the second culture container, and transferring the culture medium from the culture medium container to the second culture container.

2. The liquid delivery method for the cell culture system according to claim 1,
wherein the transfer of the cell suspension from the first culture container to the second culture container is performed via the culture container connecting tube connected to the transfer tube on the side of the culture medium container of the pump, and
the transfer of the culture medium from the culture medium container to the second culture container is performed via the culture container connecting tube connected to the transfer tube on the side of the first culture container of the pump.

3. The liquid delivery method for the cell culture system according to claim 1 or 2,
wherein the second culture container comprises two ports for liquid delivery, the connection of the second culture container and the transfer tube is performed by using two culture container connecting tubes, one side end portions of the culture container connecting tubes are connected to the two ports respectively, and the other side end portions of the culture container connecting tubes are connected to the transfer tube on both the sides of the pump.

4. The liquid delivery method for the cell culture system according to claim 1 or 2,
wherein the second culture container comprises one port for liquid delivery, the culture container connecting tube has a shape branched into two ways, and the connection of the second culture container and the transfer tube is performed by connecting one end portion of the culture container connecting tube to the one port and connecting the other end portions of the culture container connecting tubes to the transfer tube on both the sides of the pump.

5. The liquid delivery method for the cell culture system according to any one of claims 1 to 4,
wherein the cell culture system comprises at least one culture medium container.

6. The liquid delivery method for the cell culture system according to any one of claims 1 to 5,
wherein the cell culture system comprises at least one collecting container to collect the cell suspension, and
the transfer of the cell suspension from the second culture container to the collecting container is performed, by the pump, via the culture container connecting tube connected to the transfer tube on the side of the first culture container of the pump.

7. The liquid delivery method for the cell culture system according to any one of claims 1 to 6,
wherein a flow channel from each container is closed with a closing member, and in performing the liquid delivery between the respective containers, the closing member for the two containers between which the liquid delivery is performed is only removed to open the flow channel, and the liquid delivery by the pump is performed.

8. The liquid delivery method for the cell culture system according to any one of claims 1 to 7,
wherein the first culture container is an activating culture container for use in the cell culture to activate the cells, and the second culture container is a proliferating culture container for use in the cell culture to proliferate the cells.

9. A cell culture system comprising a culture medium container to supply a culture medium to a culture container, a first culture container for use in cell culture, a second culture container for use in cell culture, and a transfer tube to connect these containers,
wherein the respective containers in the cell culture system are connected to the transfer tube in the order of the culture medium container, the second culture container and the first culture container,
the transfer tube comprises only one pump, and
the second culture container is connected to the transfer tube on both sides of the pump by culture container connecting tubes to connect the second culture container and the transfer tube.

10. The cell culture system according to claim 9,
wherein the second culture container comprises two ports for liquid delivery, the connection of the second culture container and the transfer tube is performed by using two culture container connecting tubes, one side end portions of the culture container connecting tubes are connected to the two ports respectively, and the other side end portions of the culture container connecting tubes are connected to the transfer tube on both the sides of the pump.

11. The cell culture system according to claim 9,
wherein the second culture container comprises one port for liquid delivery, the culture container connecting tube has a shape branched into two ways, one end portion of the culture container connecting tube is connected to the one port, and the other end portions of the culture container connecting tubes are connected to the transfer tube on both the sides of the pump.

12. The cell culture system according to any one of claims 9 to 11,
wherein the pump is a tube roller pump.

13. The cell culture system according to any one of claims 9 to 12,
wherein a port for liquid delivery of the first culture container is disposed on the side of the transfer tube in a peripheral edge portion of the first culture container and most apart from the second culture container.

14. The cell culture system according to any one of claims 9 to 13,
wherein one of ports for liquid delivery of the second culture container is disposed on the side of the transfer tube in a peripheral edge portion of the second culture container and most apart from the culture medium container.
